# EUROPEAN PATENT SPECIFICATION

(11) **EP 2 065 680 B1**
(45) Date of publication and mention of the grant of the patent: **06.03.2013**
(21) Application number: 08169633.8
(22) Date of filing: 21.11.2008
(51) Int. Cl.: G01C 22/00, A61B 5/22, A61B 5/11

(54) **Body Movement Detecting Apparatus**
Vorrichtung zur Erfassung von Körperbewegungen
Appareil de détection des mouvements du corps

(30) Priority: 30.11.2007 JP 2007310800
(43) Date of publication of application: 03.06.2009
(73) Proprietor: Tanita Corporation, Tokyo (JP)
(72) Inventor: Nishibayashi, Kenji, Tokyo (JP)
(74) Representative: Haley, Stephen

(56) References cited:
- JP-A- 2002 190 007
- KARANTONIS D M ET AL: "Implementation of a real-time human movement classifier using a triaxial accelerometer for ambulatory monitoring" IEEE TRANSACTIONS ON INFORMATION TECHNOLOGY IN BIOMEDICINE, vol. 10, no. 1, January 2006 (2006-01), pages 156-167, XP002593105 IEEE USA ISSN: 1089-7771
- CROUTER S E, CLOWERS K G, BASSETT D R: "A novel method for using accelerometer data to predict energy expenditure" JOURNAL OF APPLIED PHYSIOLOGY, vol. 100, 1 December 2005 (2005-12-01), pages 1324-1331, XP002593106 ISSN: 8750-7587 DOI: 10.1152/japplphysiol.00818.2005

## Description

The present invention relates to a body movement detecting apparatus which detects a body movement of an user and calculates energy consumed by the body movement.

In the related art, there are pedometers as one of the body movement detecting apparatus, and specifically those having a consumption energy calculating function added thereto are widely used. Such pedometers are adapted to count the number of steps made by the walking exercise of the user, and calculate the consumption energy according to the number of steps. As other types of body movement detecting apparatus, for example, the one disclosed in JP-A-2002-191580 is proposed.

However, even when the user makes some movements, the pedometer in the related art cannot calculate the consumption energy consumed by such movements unless it is not counted as the steps made by the walking exercise of the user. The period that a person make an exercise harder than the walking exercise per day might not be much in many cases, and hence if the accurate calculation of the consumption energy relating to the body movement from which the number of steps is not counted, or is hardly counted (for example, house work such as cleaning of the house or gardening) is not achieved, the total consumption energy of the user cannot be seen accurately.

As shown in JP-A-2002-191580, even when a plurality of body movement sensors whose direction of movement detected thereby is different from each other are arranged, signals other than signals generated by the walking exercise are eliminated from output signals from the body movement sensors (see paragraph [0037]), the output signals are eventually not more than representing the walking exercise as the body movement, and hence the above-described problem is not solved.

In this manner, in the body movement detecting apparatus in the related art, the body movement to be detected is needed to be the walking exercise, and hence the user cannot calculate the energy that the user consumes in the action including not only the walking exercise, but also other daily exercises totally. Therefore, the apparatus cannot be considered to be sufficient for other objects such as daily health care administration, dieting, and so on.

"Implementation of a real-time human movement classifier using a triaxial accelerometer for ambulatory monitoring", IEEE Transactions on Information Technology in Biomedicine, vol. 10, number 1, January 2006, pages 156 to 167, and "Another method for using accelerometer data to predict energy expenditure", journal of Applied Physiology, vol. 100 December 2005, pages 1324 to 1331 both disclosed body movement detection devices.

In view of such problems, it is an object of the invention to provide a body movement detecting apparatus which is able to discriminate a walking exercise and exercises other than the walking exercise and calculate consumption energies according to the mode of the exercise, so that an energy consumed by an user including the energy consumed by the exercises other than the walking exercise is calculated totally accurately.

In order to solve the above-described problem, the present invention provides a body movement detecting apparatus comprising:
a body movement data acquiring unit that acquires body movement data relating to a body movement of an user which is generated by moving the body movement detecting apparatus for discriminating of the body movement of the user;
a body movement discriminating unit that discriminates whether the body movement is a continuous walking exercise or an exercise other than the continuous walking exercise on the basis of the body movement data; and
a computing unit arranged to
calculate a consumption energy during the walking exercise using a first calculation formula on the basis of the body movement data of the body movement which is discriminated as the continuous walking exercise from among the body movement data by the body movement discriminating unit, the first calculation formula being a formula for calculating the consumption energy consumed by the walking exercise,
calculate a consumption energy at the time of exercise other than the continuous walking exercise using a second calculation formula on the basis of the body movement data of the body movement which is discriminated as the exercise other than the continuous walking exercise from among the body movement data by the body movement discriminating unit, the second calculation formula being a formula for calculating the consumption energy consumed by the exercises other than the walking exercise, and
calculate a consumption energy by the body movement of the user by adding these consumption energy from among the body movement data by the body movement discriminating unit;
wherein the body movement data includes a difference between an upper peak value and a lower peak value of a body movement strength at every certain elapsed time and the body movement pitch, and
the body movement discriminating unit discriminates the body movement of the user as being a walking when the difference between an upper peak value and a lower peak value of the body movement strength at every certain elapsed time exceeds a first threshold value defined as a threshold value of the difference between an upper peak value and a lower peak value of the body movement strength at every certain elapsed time within a first predetermined time period, and discriminates the body movement of the user as being the continuous walking exercise when the number of times the body movement discriminating unit discriminates the body movement of the user as being a walking within the a second predetermined time period exceeds a second threshold value defined as a threshold value of the number of times the body movement discriminating unit discriminates the body movement of the user as being a walking within a second predetermined time period.

In the body movement detecting apparatus, the body movement data includes acceleration values generated by the body movement, and the body movement discriminating unit includes an accelerator sensor which outputs different output values according to the acceleration values.

Preferably, a biological data acquiring unit that acquires a biological data of the user is provided, and the computing unit calculates the consumption energy consumed by the body movement of the user using a calculation formula having the biological data acquired by the biological data acquiring unit and the body movement data as parameters.

Preferably, calculation of the consumption energy during the walking exercise is achieved by using a calculation formula including parameters at least such as a body weight as the biological data and a coefficient and a number of steps corresponding to the body movement pitch of the user as the body movement data.

Preferably, calculation of the consumption energy during exercises other than the walking exercise is achieved by using a calculation formula including parameters at least such as a body weight and a lean body mass as the biological data and data relating to acceleration values generated by the body movement as the body movement data.

Preferably, the consumption energy consumed by the body movement of the user is calculated assuming that a running exercise is included in the walking exercise.

According to the invention, not only the energy consumed by the walking exercise, but also the energy consumed by the exercises other than the walking exercise can be calculated, so that the energy consumed by the user is calculated totally and accurately.

**In the Drawings;**
Fig. 1 is a block diagram showing a configuration of a body movement detecting apparatus according to a first embodiment of the invention;
Fig. 2 is a graph showing an example of the result of measurement of a body movement data by the body movement detecting apparatus according to the first embodiment of the invention;
Fig. 3 is a flowchart showing an example of a flow of operation of the body movement detecting apparatus according to the first embodiment of the invention;
Fig. 4 is a graph showing an example of the result of measurement of the body movement data by the body movement detecting apparatus according to a second embodiment of the invention;
Fig. 5 is a flowchart showing an example of a flow of operation of the body movement detecting apparatus according to the second embodiment of the invention;

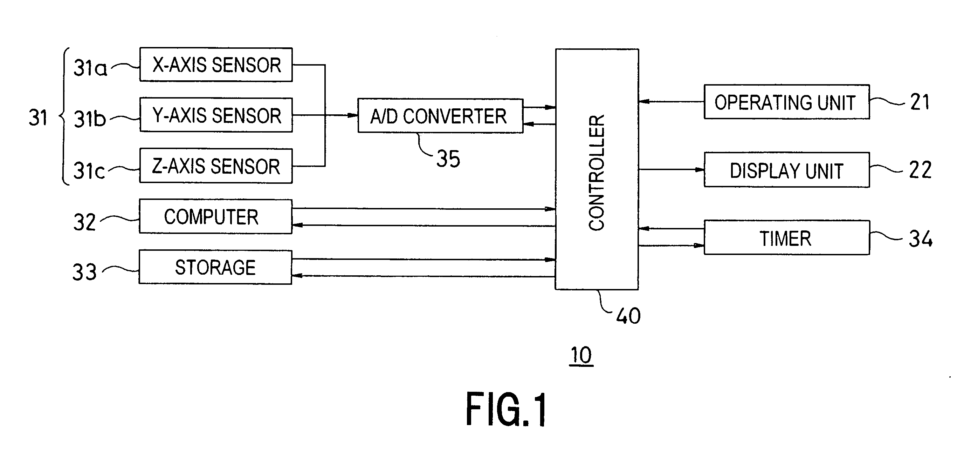

Referring now to the drawings, a body movement detecting apparatus according to a first embodiment of the invention will be described. Fig. 1 is a block diagram showing a configuration of a body movement detecting apparatus 10. As shown in Fig. 1, the body movement detecting apparatus 10 according to the first embodiment includes an operating unit 21, a display unit 22, an accelerator sensor 31, a computer 32, a storage 33, a timer 34, an A/D converter 35, and a controller 40. Configurations of respective components will be described in detail below.

The operating unit 21 (biological data acquiring unit) mainly functions as data input unit for entering a biological data of an user or for entering set items of the body movement detecting apparatus 10. The number, the shape, and the controlling method of the operating unit 21 are not specifically limited, and may be selected as needed from a button type, a touch sensor type, a dialing type, and so on. The biological data to be entered via the operating unit 21 includes, for example, the body weight, the height, the age, the sex, and the lean body mass. However, the biological data is not specifically limited as long as it is preferable biological data for obtaining a consumption energy consumed by a body movement of the user as described later. The term "preset items" means items to be set by the user when using the body movement detecting apparatus 10 and, for example, includes activation and termination of the body movement detecting apparatus 10, current date and time, and switching of the display contents displayed on the display unit 22. The biological data and the preset items entered in this manner are stored in the storage 33 (for example, RAM (Random Access Memory)) or displayed on the display unit 22 by the control of the controller 40.

The display unit 22 is a data display unit that displays data sent from the controller 40, and mainly displays the biological data of the user, the preset items, an operation guide, and consumption energy and body movement data (for example, the number of steps). The term "body movement data" here is data on the body movement of the user and, more specifically, data which reflects the body movement of the user (for example, a walking exercise, a running exercise, and other exercises) including the body movement data relating to the strength of the body movement (a body movement strength), repetition and continuity of the body movement, the pitch of the body movement when the same movement is repeated (a body movement pitch) and the number of times (for example, the number of steps). Data relating to acceleration values of the body movement of the user is preferably used as the body movement strength, and the acceleration values may be analogue data obtained by an acceleration measurement instrument, or may be data after having converted into digital data via analogue-digital conversion (hereinafter, referred to as an A/D conversion). The data relating to the acceleration values may be selected as needed from a value obtained by subtracting a lower peak value from an upper peak value of each body movement, the acceleration values by itself for each body movement, an integrated value of the acceleration values per a given period (the magnitude of the acceleration values, described later). The term "body movement" designates general movements of the user' s body, and includes not only the walking exercise and the running exercise, (hereinafter, these movements are referred generically to as walking exercise), but also exercises other than the walking exercise (for example, a step movement having no or extremely small extent of repetition or continuity, a movement of only the upper half body, and so on). The display contents described above is stored in the storage 33, and the controller 40 reads out data from the storage 33 according to the state of usage of the body movement detecting apparatus 10 and displays on the display unit 22 according to the program stored in the storage 33 in advance.

For example, a display unit using liquid crystal such as full-dot LCD (Liquid Crystal Display) may be employed as the display unit 22, and alternatively, the display unit 22 and the operating unit 21 may be configured integrally as a liquid crystal display panel having, for example, a touch panel function.

The body movement detecting apparatus 10 includes the accelerator sensor 31, the computer 32, the storage 33, the timer 34, the A/D converter 35, and the controller 40 as an internal mechanism. The computer 32 and the controller 40 each are preferably configured with an integrated circuit.

The timer 34 measures an elapse of a predetermined period or determines whether or not the predetermined time is elapsed. For example, it is able to measure the elapsed time from a moment when the user starts to use the body movement detecting apparatus 10 or to determine the body movement pitch of the user (for example, the time required for one step). In the first embodiment, the timer 34 is configured as an independent component. However, it may be integrated with the controller 40 as a timer circuit for determining whether the predetermined time is elapsed or not by the controller 40 by itself.

The accelerator sensor 31 is a body movement data acquiring unit that acquires the body movement data relating to the body movement of the user, and is a sensor which outputs various output values according to the acceleration values using the acceleration values generated by the body movement of the user as one of the body movement data. More specifically, the accelerator sensor 31 includes a X-axis sensor 31a, a Y-axis sensor 31b, and a Z-axis sensor 31c so as to detect the body movement in the directions of three axes which are orthogonal to each other (X-axis, Y-axis, and Z-axis) (see Fig. 1), so as to acquire a synthesized value of output values from the X-axis sensor 31a, the Y-axis sensor 31b, and the Z-axis sensor 31c as the acceleration values. In the first embodiment, since the accelerator sensor 31 is used as the body movement data acquiring unit, the body movement strength of the user is the data relating to the acceleration values, and the body movement data is acquired in such a manner that the body movement strength is determined to be heavy when the acceleration values are high, and to be light when the acceleration values are low.

The respective output values as the analogue data acquired by the X-axis sensor 31a, the Y-axis sensor 31b, and the Z-axis sensor 31c of the accelerator sensor 31 are converted into digital data respectively by the A/D converter 35 for the processing by the controller 40 or the computer 32, and is stored in the storage 33 corresponding to a predetermined elapsed time from the start of acquisition in conjunction with the timer 34. The A/D converted values of the respective output values of the X-axis sensor 31a, the Y-axis sensor 31b, and the Z-axis sensor 31c are combined by the computer 32 to obtain the acceleration values as digital data (the A/D converted value of the acceleration values) by calculation, and the digital acceleration values are stored in the storage 33 corresponding to the predetermined elapsed time from the start of acquisition in conjunction with the timer 34. In this manner, by acquiring the acceleration values corresponding to the elapsed time, not only the body movement strength, but also presence or absence of the repetition and continuity of the body movement, the pitch when the same body movement is repeated (body movement pitch), and the number of times (for example, the number of steps) are acquired simultaneously as the body movement data by observing the acceleration values in sequence of acquisition in time series. In order to acquire the acceleration values of all the body movement of the user more accurately by the accelerator sensor 31, attachment of the body movement detecting apparatus 10 to the user is preferably in tight contact with the user's body as much as possible and, specifically, a state of being attached to a belt which is put around the waist of the user or a state of being put into a chest pocket of a dressing of the user are preferably recommended so that the weight of the upper half body is detected. The body movement data acquired in this manner is stored in the storage 33 or partly (for example, the number of steps) displayed on the display unit 22 under the control of the controller 40.

As shown in Fig. 1, the controller 40 is electrically connected to the operating unit 21, the display unit 22, the accelerator sensor 31, the computer 32, the storage 33, the timer 34, and the A/D converter 35, and the operations thereof are controlled by the controller 40. The controller 40 functions as the body movement discriminating unit that discriminates whether the body movement is the walking exercise or exercises other than the walking exercise on the basis of the body movement data (the body movement strength, the body movement pitch) acquired by the accelerator sensor 31.

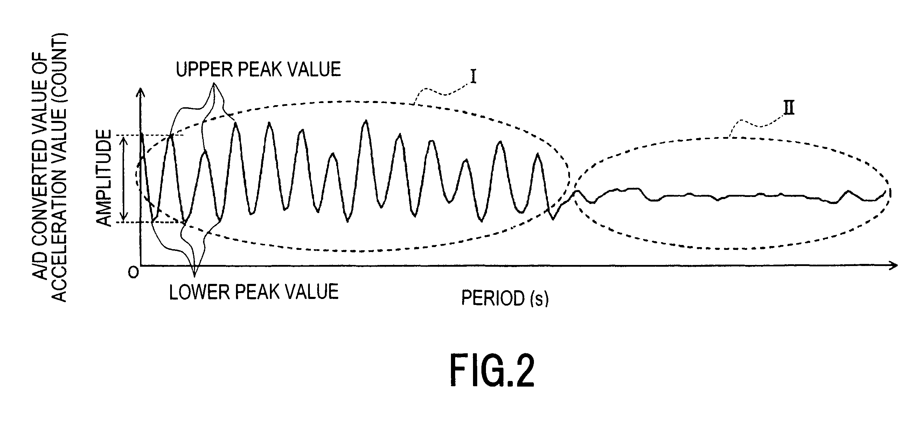

Referring now to Fig. 2, discrimination between the walking exercise and exercises other than the walking exercise will be described below. Fig. 2 is a graph showing an example of the result of acquisition of the body movement data by the body movement detecting apparatus 10.
Fig. 2 shows a state of being shifted from the walking exercise (a portion I in Fig. 2) to an exercise other than the walking exercise (a portion II in Fig. 2) as an example of the body movement of the user.

The controller 40 converts the acceleration values acquired by the accelerator sensor 31 from analogue to digital by the A/D converter 35, stores the same in the storage 33 in the time series of acquisition, acquires a waveform by plotting all the A/D converted acceleration values acquired in sequence with the elapsed time (unit: second) on the lateral axis and the A/D converted acceleration value (unit: count) on the vertical axis, and observes the transition of the acceleration values for the determination. In the process of acquiring the waveform, the acceleration values as the analogue data acquired by the accelerator sensor 31 may be plotted without processing, or the value after having applied a certain process to the A/D converted acceleration values for simplifying the observation of the waveform may be plotted.

Discrimination between the walking exercise and exercises other than the walking exercise is performed by the controller 40 according to a program stored in the storage 33 in advance. In the discrimination, a first threshold value X and a second threshold value Y set in advance and stored in the storage 33 may be used. The first threshold value X is a threshold value of the difference (amplitude) between the upper peak value and the lower peak value of the acceleration values and, for example, a value having an adequate wave amplitude for the determination of one step of the walking exercise is set (see Fig. 2). Furthermore, since whether or not the upper peak value and the lower peak value of the acceleration values are adequate as the time required for one step of the walking exercise, it is preferable to set a given time t1. The second threshold value Y is a threshold value of the number of times of the body movement which is determined as walking within a given period t2 and, for example, the number of waves (one cycle) shown by the acceleration values adequate for determining the body movement to be the continuous walking exercise is set. The value of the given period t2 for counting the number of waves in the waveform of the acceleration values in the case of determining whether or not the body movement is the continuous walking exercise may be set as needed to a value adequate for confirming that it is a continuous movement of the walking exercise.

In this manner, the two threshold values (the first threshold value X and the second threshold value Y) are set, so that the body movement is the walking exercise or an exercise other than the walking exercise can be determined generally as follows. In the first embodiment, the first threshold value X and the second threshold value Y are used as the threshold values. However, the details and the number of threshold values are not limited specifically as long as whether or not the body movement is the walking exercise or an exercise other than the walking exercise can be determined, and may be set as needed.
(1) In the waveform of the acceleration values obtained in time sequence, whether or not the amplitude can be determined as one step of the walking exercise (whether or not the difference between the upper peak value and the lower peak value exceeds the first threshold value X, and whether or not the upper peak value and the lower peak value are acquired within the given period t1) is observed. When the predetermined amplitude cannot be obtained, the body movement is determined to be an exercise other than the walking exercise.
(2) When the amplitude of the waveform of the acceleration values exceeds the first threshold value X and is acquired within the given period t1, whether or not the number of waves in the waveform of the acceleration values within the given period t2 exceeds a predetermined number (second threshold value Y) and, when it is the predetermined number of waves or smaller, the body movement is determined as an exercise other than the walking exercise and, when it exceeds the predetermined number of waves, it is determined as a continuous walking exercise.

The exercises other than the walking exercise includes heavy exercises and light exercises in strength. In order to calculate the consumption energy consumed by the body movement of the user precisely, it is preferable to determine the exercises other than the walking exercise on the basis of the body movement strength, and use different energy calculation formulas depending on the result of determination. In the first embodiment, the strength of the exercise other than the walking exercise using a third threshold value Z of the body movement strength is determined.

The computer 32 (computing unit) calculates the consumption energy consumed by the body movement of the user under the control of the controller 40 on the basis of the biological data or the body movement data of the user stored in the storage 33. At this time, the computer 32 calculates the consumption energy consumed by the walking exercise on the basis of the body movement data (the body movement pitch or the number of steps) of the body movement determined as the walking exercise by the controller 40 as the body movement discriminating unit, calculates the consumption energy consumed by the exercise other than the walking exercise on the basis of the body movement data (the body movement strength) of the body movement determined as the exercise other than the walking exercise, and adds up these consumption energies, so that the total consumption energy consumed by the body movement of the user is calculated. Consumption energy calculation formulas corresponding to the walking exercise or the exercises other than the walking exercise are stored in the storage 33, respectively in advance. Consumption energy calculation formulas corresponding to the heavy exercise and the light exercise in body movement strength in the exercises other than the walking exercise are stored in the storage 33 in advance. The computer 32 calculates the consumption energy consumed by the walking exercise and the consumption energy consumed by the exercises other than the walking exercise are calculated respectively using the calculation formulas corresponding to the respective exercises selected by the controller 40, and adds up the consumption energy consumed by the walking exercise and the consumption energy consumed by the exercises other than the walking exercise, so that the consumption energy on the basis of the entire body movement of the user is calculated.

The calculation formula for calculating the consumption energy consumed by the walking exercise is, for example, "weight of the user × number of steps × coefficient". The coefficient may be set arbitrarily as a product of a constant defied according to the body movement pitch and a coefficient defined by the body movement strength. The constant which is defined by the body movement pitch may be determined, for example, to be c1 when the time required for one step (body movement pitch) is in the range from 250 ms inclusive to 300 ms exclusive, and to be c2 when it is within the range from 300 ms inclusive to 350 ms exclusive. In the same manner, the coefficient may be set so as to be increased in sequence every 50 ms (for example, c1<c2<...). On the other hand, the coefficient which is defined by the body movement strength may be a coefficient defined by data on the measured acceleration values and, for example, may be set to be increased in sequence according to the stages of the "magnitude of acceleration value", which is classified into given number of stages in advance, from a stage having a small "magnitude of acceleration value" to a stage having a large "magnitude of acceleration value", described later (for example, a1<a2<...).

By setting the coefficient as described above, calculation of the consumption energy is achieved using the same calculation formula irrespective of whether the body movement is the running exercise or the walking exercise. For example, when the body movement is the running exercise, the time required for one step (body movement pitch) is shorter than the case of the walking exercise. Therefore, on the basis of the setting of the coefficient, the coefficient defined by the body movement pitch is increased. In contrast, when the body movement is the running exercise, the acceleration value is large, and hence the coefficient defined by the body movement strength becomes large. Therefore, since the coefficient in the calculation formula of the consumption energy is large, it is determined that a larger energy than the walking exercise is consumed. By setting a given period t3 (for example, 10 seconds) as a unit time for calculating the consumption energy, the computer 32 determines to which one of the coefficients (c1, c2, ...) the average value of the body movement pitches taken at every given period t3 belongs and defines the coefficient and, simultaneously, determines to which one of the coefficients (a1, a2, ...) the average value of the body movement strengths belongs, and defines the coefficient, whereby the "consumption energy consumed by the walking exercise" generated during the given period t3 on the basis of these coefficients. Also, the "consumption energy consumed by the walking exercise" corresponding to the entire period of the walking exercise is calculated by adding up all the consumption energies consumed during each given period t3 obtained in this manner. It is also possible to adapt the apparatus to calculate instantaneous consumption energies generated in one step (the body movement data) or to add up these consumption energies to calculates the "consumption energy consumed by the walking exercise" corresponding to the entire number of steps.

In contrast, the calculation formula for calculating the consumption energy consumed by the exercises other than the walking exercise is, for example, "weight of the user × magnitude of acceleration value × first coefficient + second coefficient". The first coefficient and the second coefficient may be set arbitrarily. However, it is preferable to set these coefficients to different values according to the sex (biological data). When the height and the lean body mass may be used as the biological data of the user in addition to the sex, further accurate consumption energy is calculated by introducing terms including such data. The calculation formula in this case is preferably prepared separately for each sex. For example, a calculation formula "acceleration value (or acceleration value applied with certain processing) × body weight × third coefficient + lean body mass × fourth coefficient - fifth coefficient" is employed as the formula for male and a calculation formula "acceleration value (or acceleration value applied with certain processing) × body weight × sixth coefficient (lean body mass/square of height) × seventh coefficient - eighth coefficient" is employed as the formula for female. In this case as well, the third coefficient to the eighth coefficient may be set arbitrarily. However, it is preferable to set these coefficients to different values according to the sex (biological data). In this manner, the computer 32 calculates the "consumption energy consumed by the exercises other than the walking exercise" generated during the given period t3. Also, the "consumption energy consumed by the exercises other than the walking exercise" corresponding to the entire period of the exercises other than the walking exercise is calculated by adding up all the consumption energies consumed during each given period t3 obtained in this manner. The given period t3 is a unit time for calculating the consumption energy. However, it is also possible to define a given period t3₁ as the unit time for calculating the consumption energy for the "consumption energy consumed by the walking exercise" described above and define a given period t3₂ as the unit time for calculating the consumption energy for the "consumption energy consumed by the exercises other than the walking exercise" separately, or to define the same unit time as needed.

When classifying the exercises other than the walking exercise into hard exercises and light exercise in terms of the strength of the body movement, it is preferably to prepare two types of the above-described coefficients according to the body movement strength (acceleration values) and to prepare two or more calculation formulas such as a calculation formula to be used for the hard exercises (calculation formula 1 for the exercises other than the walking exercise) and a calculation formula to be used for the light exercises (calculation formula 2 for the exercises other than the walking exercise) according to the body movement strength from among the exercises other than the walking exercise. Accordingly, when the average value of the acceleration values measured during the given period t3 is the third threshold value Z or larger, it is determined as the heavy exercise whose body movement strength is high, and when it is smaller than the third threshold value Z, it is determined as the light exercise whose body movement strength is low, so that calculation of the consumption energy is achieved using the corresponding calculation formulas respectively.

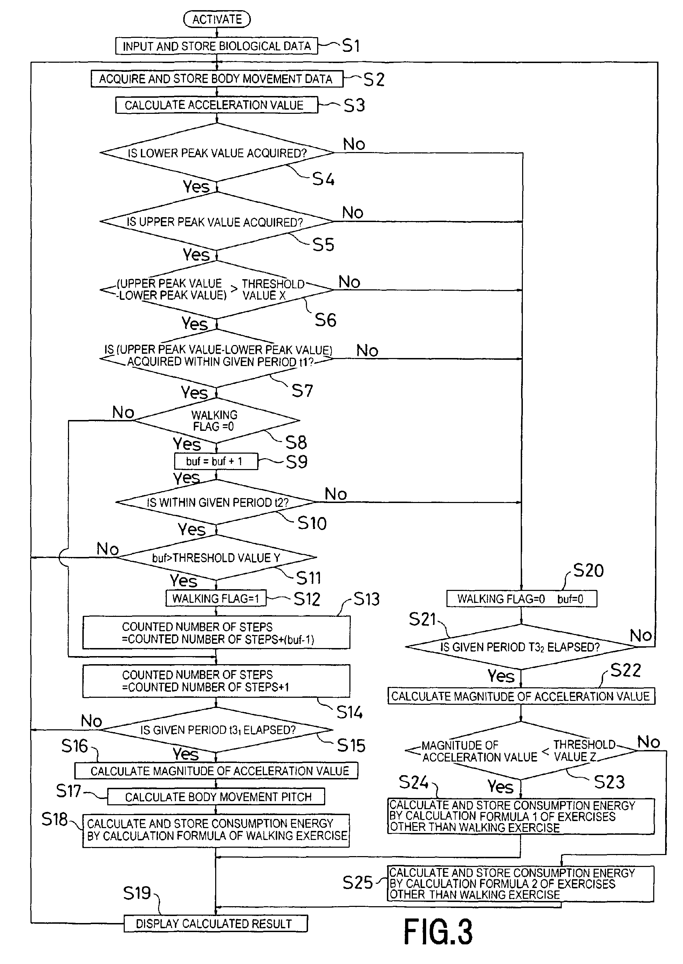

Referring now to Fig. 3, the calculation of the consumption energy by the body movement detecting apparatus 10 will be described. Fig. 3 is a flowchart showing an example of a flow of operation of the body movement detecting apparatus 10.

After having activated the body movement detecting apparatus 10 and prior to the acquisition of the body movement data, the user operates the operating unit 21 and enters the biological data and the set items, whereby the entered biological information and the set items are stored in a predetermined area in the storage 33, so that the initial setting is achieved (Step S1). The stored biological data and the set items can be read out by operating the operating unit 21 and displayed on the display unit 22, so that the user is able to make a correction by operating the operating unit 21 as needed while viewing the display. After having completed such the input operation, the body movement detecting apparatus 10 is attached to a predetermined position such as on the dressing of the user.

Acquisition of the body movement data by the accelerator sensor 31 of the body movement detecting apparatus 10 is started, and the acquired body movement data is stored into the storage 33 (Step S2) . More specifically, the A/D converter 35 converts output values from the accelerator sensor 31 acquired by the X-axis sensor 31a, the Y-axis sensor 31b, and the Z-axis sensor 31c as analogue data into digital data respectively, and the controller 40 acquires an elapsed time from a point when the acquisition is started (or a current time) by the timer 34 at the same time, and stores the A/D converted value of the respective output values in the storage unit corresponding to the elapsed time from the point when the acquisition is started (or the current time).

Then, the computer 32 combines the A/D converted values of the respective output values from the X-axis sensor 31a, the Y-axis sensor 31b, and the Z-axis sensor 31c to obtain the acceleration value as digital data (the A/D converted value of the acceleration value) by calculation, and the controller 40 stores the A/D converted values of the acceleration values corresponding to the elapsed time (Step S3).

More specifically, waveforms are acquired respectively for the output value from the X-axis sensor 31a, the output value from the Y-axis sensor 31b, and the output value from the Z-axis sensor 31c with the lateral axis representing the elapsed time (unit: second) and the vertical axis representing the A/D converted values of the acceleration value (unit: count). Subsequently, n samples are extracted respectively from output values of the X-axis sensor 31a (X₁, X₂, ... Xₙ), output values of the Y-axis sensor 31b (Y₁, Y₂, ... Yₙ), and output values of the Z-axis sensor 31c (Z₁, Z₂, ... Zₙ) at every given period t4, and average values (AX, AY, AZ) are calculated. The given period t4 is a time interval for calculating the average values, and the value n is the number of samples for calculating these average values, and these values may be set as needed. When the larger number of n is set, the average value is based on the larger number of data, so that calculation of the consumption energy with high degree of accuracy is advantageously achieved.

Subsequently, the absolute values from the average values are obtained for the respective samples, and combined to calculate the "acceleration value". For example, as regards the first sample, that is, the output value X₁ of the X-axis sensor 31a, the output value Y₁ of the Y-axis sensor 31b, and the output value Z₁ of the Z-axis sensor 31c, the acceleration value of the first sample is calculated using the respective average values AX, AY, and AZ with an expression √((X₁-AX)² + (Y₁-AY)² + (Z₁-AZ)²). In the same manner, the acceleration values are calculated to the n^{th} sample, and this calculation is repeated to obtain the acceleration values, and then all the acceleration values calculated in sequence are plotted with the lateral axis representing the elapsed time (unit: second) and the vertical axis representing the A/D converted value of the acceleration value (unit: count), so that the waveform of the acceleration value is acquired.

In the waveform of the acceleration value (see Fig. 2), whether or not the lower peak value is acquired (Step S4) and whether or not the upper peak value is acquired (Step S5) are determined in sequence. When the lower peak value is not acquired (No in Step S4), or when the upper peak value is not acquired (No in Step S5), the walking flag is set to zero, and it is determined that the exercises other than the walking exercise is being performed (Step S20).

In contrast, when the lower peak value is acquired (Yes in Step S4) and also the upper peak value is acquired (Yes in Step S5), the difference between the acquired upper peak value and the lower peak value (amplitude) is calculated. Whether or not the difference between the upper peak value and the lower peak value exceeds the first threshold value X is determined (Step S6) and, when it does not exceed the first threshold value X (No in Step S6), the walking flag is set to zero, and it is determined that the exercises other than the walking exercise is being performed (Step S20).

When the difference between the upper peak value and the lower peak value exceeds the first threshold value X (Yes in Step S6), whether or not the difference between the upper peak value and the lower peak value is acquired within the given period t1 is determined (Step S7). When it is considered that the amplitude value is not acquired within the given period t1 (No in Step S7), the walking flag is set to zero and it is determined that the exercises other than the walking exercise is being performed (Step S20).

On the other hand, when the difference between the upper peak value and the lower peak value is acquired in the given period t1 (Yes in Step S7), whether or not the walking flag is zero is determined (Step S8). When the walking flag is zero (Yes in Step S8), the buffer corresponding to the walking flag is added by one (Step S9). The buffer is data on the number of times of body movements stored temporarily for determining that the body movement of the user is the walking exercise and is the number of waves of the waveform of the acceleration value, that is, the number of steps which satisfies the conditions in Step S6 and Step S7 in the first embodiment.

When the value of the buffer exceeds the given period t2 from the time point when the value of the buffer becomes 1 in Step S9 (No in Step S10), the walking flag is set to zero and the value of the buffer is reset to zero, and it is determined that the exercises other than the walking exercise is being performed (Step S20). In contrast, when it is within the given period t2 from the time point when the value of the buffer becomes 1 in Step S9 (Yes in Step S10), whether or not the value of the buffer is larger than the second threshold value Y is determined (Step S11). Accordingly, when the value of the buffer exceeds the second threshold value Y (for example, 10 steps) in the given period t2 (for example, 10 seconds), it is determined that the user is performing the continuous walking exercise. In this manner, the given period t2 and the second threshold value Y may be set to a period and the number of steps adequate for determining that the user is performing the continuous walking exercise. When the value of the buffer does not exceed the second threshold value Y (No in Step 11), the procedure goes back to Step S2 where the same process is repeated. When the value of the buffer is larger than the second threshold value Y (Yes in Step S11), the walking fag is rewritten to 1 (Step S12), then the current number of steps is rewritten by adding the value obtained by subtracting 1 from the buffer value (Step S13) and, the current number of steps is further rewritten by adding 1 to the current number of steps (Step S14). The reason why the value obtained by subtracting 1 from the buffer value is added to the counted number of steps in Step S13 is because it is necessary by right to add the number of steps (the buffer value) made immediately before determination of the start of the continuous walking exercise for making this determination and, in Step S13, the value obtained by subtracting 1 from the buffer value is added considering that the counted number of steps is further added by 1 in Step S14. As described above, when the walking flag is set to 1 and it is determined that the continuous walking exercise is started, the steps from Step S9 to Step S13 are omitted and the procedure goes from Step S8 to Step S14. In this manner, whether or not it is the walking exercise is determined on the basis of the consideration of the buffer value, the reason being because there is a case where the user simply makes a discontinuous body movement which cannot be determined as the continuous walking exercise even when the user does several steps, and hence the body movement such as the steps which cannot be determined as the continuous walking exercise but are determined as a noise should be excluded from the walking exercise. However, the energy consumed by such the movement excluded from the walking exercise can also be calculated as described later, and hence the entire energy consumed by the user including the consumption energy consumed by the exercises other than the walking exercise is calculated totally and adequately.

Subsequently, whether the given period t3₁, which is a unit time for calculating the consumption energy, has elapsed or not is determined (Step S15). Here, the starting point of calculation of the given period t3₁ is set as an example shown below.
(1) When the given period t3₁ is the unit time to which the time point when it is determined that the continuous walking exercise is started belongs (the time point where the walking flag is set to 1), the time point of the first step of the continuous walking exercise is set as the starting point of calculation. Therefore, in Fig. 3, in the cases where (a) the determination in Step S8 is Yes, and the procedure goes to Step S15 via the Step S9 to Step S13, (b) the determination in Step S8 is No, and the procedure omits Step S9 to Step S13 and goes to Step S15, and the given period t3₁ belongs to the same unit time as (a) describe above, the time point when "buf=1" in Step S9 is set to the starting point of calculation.
(2) When the given period t3₁ is a unit time after the unit time to which the time point when it is determined that the continuous walking exercise is started belongs (the time point where the walking flag is set to 1), the time point when the unit time immediately before is ended is set as the starting point of calculation. Therefore, in Fig. 3, the starting point of calculation of the unit time in the case where the procedure goes to Step S15 after having calculated the consumption energy at least once in Step S18 is set to the time point when the unit time immediately before is ended.

When it is before having elapsed the given period t3₁ (No in Step S15), the procedure goes back to Step S2, and the same process is repeated. In contrast, when the given period t3₁ is elapsed (Yes in Step S15), the controller 40 and the computer 32 calculate the "magnitude of the acceleration values" (the body movement strength) of the walking exercise during the given period t3₁ (Step S16), determine predetermined coefficient corresponding to the respective values, and store the same in the storage 33. Here, the magnitude of the acceleration value is a product of the acceleration values (the respective plotted values in Step S3) during the given period t3₁ as the unit time for calculating the consumption energy.

The controller 40 and the computer 32 calculate the average value of the body movement pitch by the walking exercise during the given period t3₁ (Step S17), determine predetermined coefficient corresponding to the respective values, and store the same in the storage 33. Calculation of the average value of the body movement pitch is achieved, for example, by obtaining the average value of the intervals (time) between the upper peaks (or the lower peaks) of the respective waves (respective numbers of steps) in the waveform of the acceleration value during the given period t3₁.

The computer 32 applies a coefficient determined by the body weight, the number of steps, the body movement pitches, and the body movement strength of the user to the calculation formula for calculating the consumption energy consumed by the walking exercise to calculate the "consumption energy consumed by the walking exercise" during the given period t3₁ (Step S18) . At this time, it also calculates "the consumption energy consumed by the walking exercise" corresponding to the entire period of the walking exercise by adding up all the consumption energies consumed during each given period t3₁ obtained in this manner. The controller 40 stores "the consumption energy consumed by the walking exercise" calculated in this manner in the storage 33.

When the body movement is determined to be an exercise other than the walking exercise (Step S20), the walking flag is set to zero, and is determined that the exercise other than the walking exercise is started, and then whether or not the given period t3₂ as the unit time for calculating the consumption energy has elapsed is determined (Step S21) . Here, the starting point of calculation of the given period t3₂ is set as an example shown below.
(1) When the given period t3₂ is a unit time to which the time point when the first body movement data is acquired in Step S2 immediately after Step S1 belongs, the corresponding point is determined to be the starting point of calculation.
(2) When the given period t3₂ is a unit time to which the time point when the body movement is shifted from the walking exercise to the exercises other than the walking exercise (the time point when the walking flag is rewritten from 1 to 0) belongs, the time point when the unit time (given period t3₁) for calculating the consumption energy of the walking exercise done immediately before is ended is set to the starting time of calculation.
(3) When the given period t3₂ is a unit time after the unit time in (1) or (2) described above, the time point when the unit time immediately before is ended is set to the starting point of calculation.

When it is before having elapsed the given period t3₂ (No in Step S21), the procedure goes back to Step S2, and the same process is repeated. In contrast when the given period t3₂ has elapsed (Yes in Step S21), the "consumption energy consumed by the exercises other than the walking exercise" is calculated. In the first embodiment, an example in which the consumption energy with a higher degree of accuracy can be calculated by determining whether the exercise other than the walking exercise is a heavy exercise or a light exercise and using different calculation formulas depending on the strength will be described.

The controller 40 and the computer 32 calculate the "magnitude of the acceleration value" (the body movement strength) in the exercises other than the walking exercise during the given period t3₂ (Step S22). Here, the magnitude of the acceleration value is a product of the acceleration values (the respective plotted values in Step S3) during the given period t3₂ as the unit time for calculating the consumption energy.

Then, when the magnitude of the acceleration value in the given period t3₂ (the body movement strength) is smaller than the third threshold value Z (Yes in Step S23), the controller 40 and the computer 32 select a calculation formula used in the case of the light exercise from among the exercises other than the walking exercise (a calculation formula 1 for the exercises other than the walking exercise) to calculate the "consumption energy consumed by the exercises other than the walking exercise" during the given period t3₂ (Step S24), and when it is third threshold value Z or larger (No in Step S23), the controller 40 and the computer 32 select a calculation formula used in the case of the heavy exercise from among the exercises other than the walking exercise (a calculation formula 2 for the exercises other than the walking exercise) to calculate the "consumption energy consumed by the exercises other than the walking exercise" during the given period t3₂ (Step S25) . At this time, the computer 32 may calculate the "consumption energy consumed by the exercises other than the walking exercise" corresponding to the entire period of the exercises other than the walking exercise which is calculated by adding up all the consumption energies consumed during each given period t3 calculated in the same manner. The controller 40 stores "the consumption energy consumed by the exercises other than the walking exercise" calculated in this manner in the storage 33.

The computer 32 adds up the "consumption energy consumed by the walking exercise" and the "consumption energy consumed by the exercises other than the walking exercise" calculated in this manner as needed to calculate the entire consumption energy consumed by the body movement of the user, and the controller 40 displays the calculated result on the display unit 22 (Step S19). Subsequently, the procedure goes back to Step S2 to repeat the same process.

Referring now to Fig. 1, Fig. 4, and Fig. 5, a body movement detecting apparatus according to a second embodiment of the invention will be described. The body movement detecting apparatus according to the second embodiment includes an operating unit 21, a display unit 22, an accelerator sensor 31, a computer 32, a storage 33, a timer 34, an A/D converter 35, and a controller 40 like the body movement detecting apparatus 10 in the first embodiment, and hence the detailed descriptions on the respective component will be omitted. The body movement detecting apparatus according to the second embodiment employs a method of discrimination different from the body movement detecting apparatus 10 in the first embodiment as a method of discrimination of the walking exercise and the exercises other than the walking exercise. The method of discrimination of the walking exercise and the exercises other than the walking exercise will be described below.

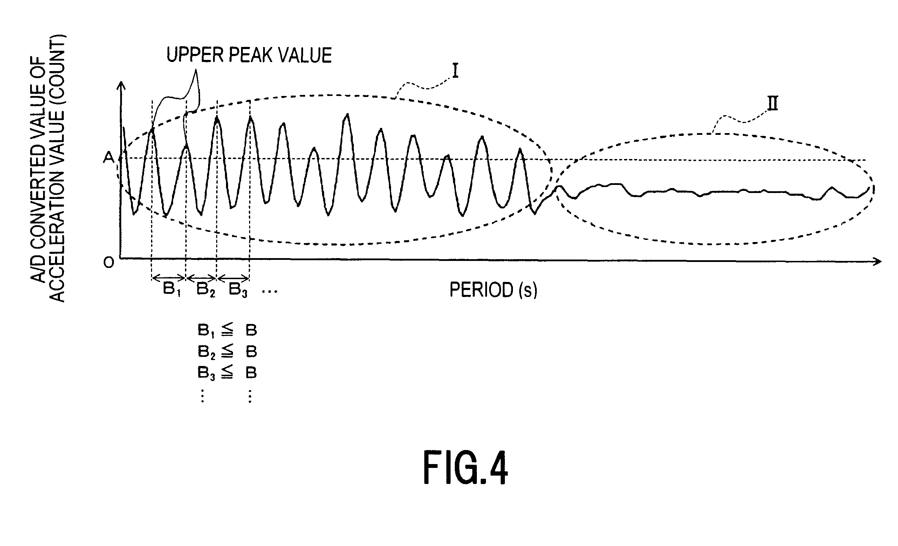

Discrimination between the walking exercise and exercises other than the walking exercise is performed by the controller 40 according to a program stored in the storage 33 in advance. In the discrimination, a fourth threshold value A and the fifth threshold value B set in advance and stored in the storage 33 may be used. The fourth threshold value A is a threshold value of the acceleration value (the body movement strength) and, an adequate acceleration value for determining the first step of the walking exercise is set (see Fig. 4). The fifth threshold value B is a threshold value of the time interval (body movement pitch) between upper peak values of adjacent waves in the waveform of the acceleration value, and a pitch per one step adequate for the determination of the walking exercise is set.

In this manner, the two threshold values (the fourth threshold value A and the fifth threshold value B) are set, so that the body movement is the walking exercise or an exercise other than the walking exercise can be determined generally as follows.
(1) In the waveform of the acceleration value acquired in time sequence, whether or not the upper peak value of a first wave has a body movement strength from which the body movement is determined to be the walking exercise (whether or not the fourth threshold value A is exceeded) is observed and, when it underruns the predetermined body movement strength, it is determined to be the exercise other than the walking exercise.
(2) When the upper peak value of the first wave exceeds the predetermined body movement strength (the fourth threshold value A), whether or not the upper peak value of a second wave following the first wave exceeds the predetermined body movement strength (fourth threshold value A) is determined and, when it underruns the predetermined body movement strength, it is determined that both the body movement corresponding to the first wave and the body movement corresponding to the second wave are the exercises other than the walking exercise.
(3) When the upper peak value of the second wave exceeds the predetermined body movement strength (fourth threshold value A), whether or not the respective upper peak values of the first wave and the second wave demonstrate the body movement pitches from which the body movement is determined to be the walking exercise (whether or not it is within the fifth threshold value B) is observed and, when they do not match the predetermined body movement pitch, it is determined to be the exercise other than the walking exercise.
(4) In the case in which the upper peak values of the first wave and the second wave demonstrate the body movement pitches from which it is determined to be the walking exercise (the case of "B₁≤ B" in Fig. 4), the waves from the third wave onward (to the N^{th} wave) are observed in the same manner and, when the predetermined body movement strength and the body movement pitches are continuously demonstrated, it is determined that the continuous walking exercise is being performed. At this time, the controller 40 serves as a step counting unit by counting the number of steps (the body movement data) by allocating one step for one wave while confirming that the wave immediately after the determination to be the walking exercise demonstrates the predetermined body movement strength and the body movement pitch, and stores the number of steps and the time required for one step (the body movement pitch) in the storage 33. On the other hand, when the predetermined body movement strength and the body movement pitch cannot be confirmed continuously, it is determined to be the exercise other than the walking exercise. When the predetermined body movement strength and the body movement pitch cannot be confirmed continuously any longer, it is determined that the body movement is shifted from the walking exercise to the exercise other than the walking exercise, and counting of the number of steps is discontinued. The value of N may be set to a value adequate for confirming that the body movement is the continuous movement, that is, the walking exercise.

The method of calculating the consumption energy consumed by the walking exercise and the consumption energy consumed by the exercises other than the walking exercise by the computer 32 is the same as in the first embodiment, and the detailed description is omitted.

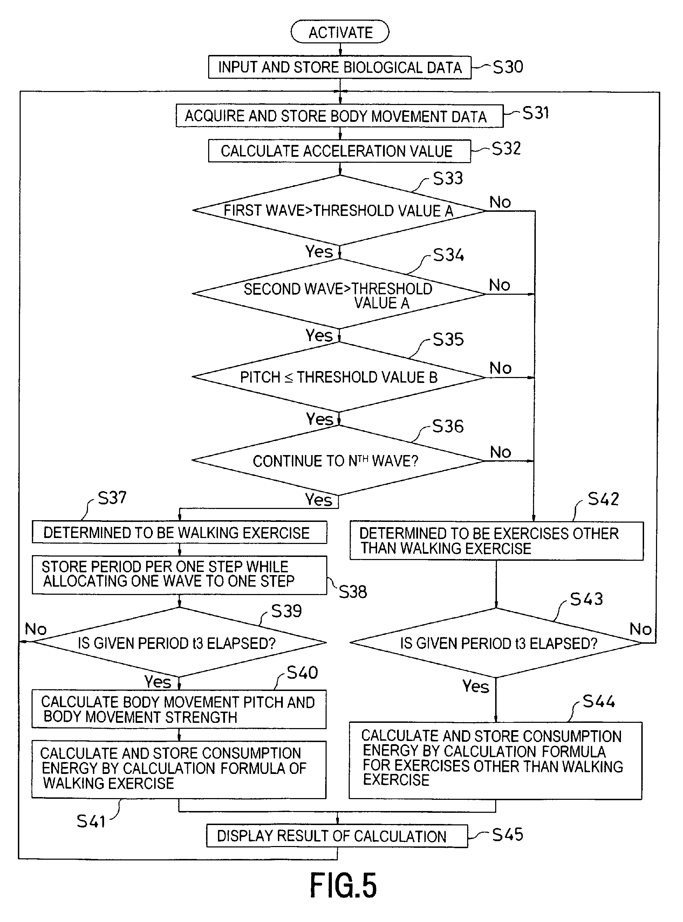

Referring now to Fig. 5, the calculation of the consumption energy by the body movement detecting apparatus 10 will be described. Fig. 5 is a flowchart showing an example of a flow of operation of the body movement detecting apparatus 10 according to the second embodiment.

The processes in Step S30 to Step S32 are the same as in the Step S1 to Step S3 in the first embodiment, and hence description is omitted. The controller 40 calculates the acceleration value (body movement data) in the same manner as in the first embodiment and, all the acceleration values calculated in sequence are plotted with the lateral axis representing the elapsed time (unit: second) and the vertical axis representing the A/D converted value of the acceleration value (unit: count) so that the waveform is acquired. In this waveform, presence or absence of the first wave having the upper peak value which exceeds the fourth threshold value A is determined (Step S33) and, while the first wave as such is not detected (No in Step S33), it is determined that the exercise other than the walking exercise is being performed (Step S42) .

When the first wave having the upper peak value exceeding the fourth threshold value A is detected (Yes in Step S33), presence or absence of the second wave having the upper peak value which exceeds the fourth threshold value A immediately after the first wave is determined (Step S34) and, while the second wave as such is not detected (No in Step S34), it is determined that the exercise other than the walking exercise is being performed (Step S42).

When the second wave having the upper peak value exceeding the fourth threshold value A is detected (Yes in Step S34), whether or not the pitch (B₁ in Fig. 4) of the respective upper peak values of the first wave and the second wave does not exceed the fifth threshold value B is determined (Step S35) and, when it exceeds the fifth threshold value B (No in Step S35), it is determined that the exercises other than the walking exercise is being performed (Step S42).

When the pitch of the respective upper peak value of the first wave and the second wave does not exceed the fifth threshold value B (B₁≤B in Fig. 4) (Yes in Step S35), whether or not the amplitudes which satisfy these conditions are generated continuously from the third wave onward (to the N^{th} wave) is determined in the same manner on the basis of the fourth threshold value A (the body movement strength) and the fifth threshold value B (the body movement pitch) (Step S36) and, when the continuous waveform cannot be observed (No in Step S36), it is determined that the exercise other than the walking exercise is being performed (Step S42). In contrast, when the fourth threshold value A (the body movement strength) and the fifth threshold value B (body movement pitch) are satisfied continuously to the N^{th} wave (Yes in Step S36), it is determined that the continuous walking exercise is started (Step S37).

The controller 40 counts the number of steps (body movement data) by allocating one step for one wave while confirming that the waves from the wave immediately after the determination of the start of the continuous walking exercise onward satisfy the fourth threshold value A (the body movement strength) and the fifth threshold value B (the body movement pitch), and stores the number of steps and the time required for one step (the body movement pitch) (Step S38). When the continuous waves which satisfy the fourth threshold value A (the body movement strength) and the fifth threshold value B (the body movement pitch) cannot be observed any longer, it is determined that the body movement is shifted from the walking exercise to the exercise other than the walking exercise, and counting of the number of steps is discontinued.

When it is before having elapsed the given period from the time point when it is determined that the continuous walking exercise is started (No in Step S39), the procedure goes back to Step S31, and the same process is repeated. The given period here corresponds to the given period t3 (or the given period t3₁) in the first embodiment. In contrast, when the given period is elapsed (Yes in Step S39), the controller 40 and the computer 32 calculate the average values of the body movement pitches and the body movement strength during the given period (Step S40), and define predetermined coefficients corresponding to the respective values and store the same in the storage 33. The average value of the upper peak values of the respective waves (respective number of steps) in the waveform of the acceleration value for the given period may be employed, for example, as the average value of the body movement strength. In contrast, the average value of the intervals between the upper peak values of the respective waves (respective number of steps) obtained in the waveform of the acceleration value during the given period may be employed as the average value of the body movement pitch.

The computer 32 applies a coefficient determined by the body weight, the number of steps, the body movement pitches, and the body movement strength of the user to the calculation formula for calculating the consumption energy consumed by the walking exercise to calculate the "consumption energy consumed by the walking exercise" during the given period, and the controller 40 stores the "consumption energy consumed by the walking exercise" calculated in this manner in the storage 33 (Step S41).

When it is determined to be the exercise other than the walking exercise (Step S42), if it is before having elapsed the given period from the time point when it is determined that the exercise other than the walking exercise is started (No in Step S43), the procedure goes back to Step S31, and the same process is repeated. The given period here corresponds to the given period t3 (or the given period t3₂) in the first embodiment. In contrast, when the given period is elapsed (Yes in Step S43), the computer 32 applies the weight of the user, the average value of the acceleration values during the given period (the body movement strength), and the coefficient defined by the biological data of the user to the calculation formula for calculating the consumption energy consumed by the exercises other than the walking exercise to calculate the "consumption energy consumed by the exercises other than the walking exercise" calculated in this manner, and the controller 40 stores the "consumption energy consumed by the exercises other than the walking exercise" in the storage 33 (Step S44).

The computer 32 adds up the "consumption energy consumed by the walking exercise" and the "consumption energy consumed by the exercises other than the walking exercise" calculated in this manner as needed to calculate the entire consumption energy consumed by the body movement of the user, and the controller 40 displays the calculated result on the display unit 22 (Step S45). Subsequently, the procedure goes back to Step S31 to repeat the same process.

The display unit 22 may be adapted to display the number of steps as the body movement data, or the consumption energies or numbers of steps of the past such as one day before, two days before, and so on in addition to the consumption energy consumed by the body movement of the user.

In this configuration, according to the first embodiment and the second embodiment, advantages as shown below are achieved.
(1) By discriminating a walking exercise and exercises other than the walking exercise and calculating consumption energies according to the mode of the exercise, an energy consumed by a user including the energy consumed by the exercises other than the walking exercise is calculated totally accurately.
(2) Since the different calculation formulas are used for the walking exercise and the exercises other than the walking exercise, and the body movement data such as the body movement strength and the body movement pitch is reflected in calculation, the consumption energy is calculated accurately according to the heaviness or the like in the exercises which are classified, for example, as the walking exercise.

The invention has been described referring to the embodiments shown above. However, the invention is not limited to the embodiments shown above, and may be improved or modified for the purpose of improvement or within the range of the scope of the invention. For example, the body movement including the walking exercise and the running exercise are defined as the "walking exercise", and the consumption energies of the walking exercise and the running exercise are obtained with the same calculation formula. However, it is also possible to prepare a calculation formula for calculating the consumption energy for the walking exercise and a calculation formula for calculating the consumption energy for the running exercise separately, determine whether or not the body movement is the walking exercise or the running exercise depending on the body movement pitch or the body movement strength of the body movement of the user, and obtain the consumption energy on the basis of the respective calculation formula.

## Claims

1. A body movement detecting apparatus comprising:
a body movement data acquiring unit arranged to acquire body movement data relating to a body movement of a user which is generated by moving the body movement detecting apparatus;
a body movement discriminating unit arranged to discriminate whether the body movement is a continuous walking exercise or an exercise other than the continuous walking exercise on the basis of the body movement data; and
a computing unit arranged to
calculate a consumption energy during the walking exercise using a first calculation formula on the basis of the body movement data of the body movement which is discriminated as the walking exercise from among the body movement data by the body movement discriminating unit, the first calculation formula being a formula for calculating the consumption energy consumed by the walking exercise,
calculate a consumption energy at the time of exercise other than the walking exercise using a second calculation formula on the basis of the body movement data of the body movement which is discriminated as the exercise other than the walking exercise from among the body movement data by the body movement discriminating unit, the second calculation formula being a formula for calculating the consumption energy consumed by the exercises other than the walking exercise, and
calculate a consumption energy by the body movement of the user by adding these consumption energies from among the body movement data by the body movement discriminating unit;
wherein the body movement data includes a difference between an upper peak value and a lower peak value of a body movement strength at every certain elapsed time and the body movement pitch, and
the body movement discriminating unit is arranged to discriminate the body movement of the user as being a walking exercise when the difference between an upper peak value and a lower peak value of the body movement strength at every certain elapsed time exceeds a first threshold value defined as a threshold value of the difference between an upper peak value and a lower peak value of the body movement strength at every certain elapsed time within a first predetermined time period, and to discriminate the body movement of the user as being the continuous walking exercise when the number of times the body movement discriminating unit discriminates the body movement of the user as being a walking within a second predetermined time period exceeds a second threshold value defined as a threshold value of the number of times the body movement discriminating unit discriminates the body movement of the user as being a walking exercise within a second predetermined time period.

2. The body movement detecting apparatus according to Claim 1 comprising an acceleration value generated by the body movement, wherein the body movement discriminating unit includes an accelerator sensor arranged to output different output values according to the acceleration value.

3. The body movement detecting apparatus according to Claims 1 or 2, further comprising a biological data acquiring unit that is adapted to acquire biological data of the user, wherein the computing unit is arranged to calculate the consumption energy consumed by the body movement of the user using a calculation formula having the biological data acquired by the biological data acquiring unit and the body movement data as parameters.

4. The body movement detecting apparatus according to Claim 3, arranged such that the calculation of the consumption energy during the walking exercise is achieved by using a calculation formula including parameters at least such as a body weight as the biological data and a coefficient and a number of steps corresponding to the body movement pitch of the user as the body movement data.

5. The body movement detecting apparatus according to Claim 3 or Claim 4, arranged such that calculation of the consumption energy during exercises other than the walking exercise is achieved by using the calculation formula including parameters at least such as the body weight and a lean body mass as the biological data and data relating to an acceleration value generated by the body movement as the body movement data.

6. The body movement detecting apparatus according to any one of Claims 1 to 5, arranged such that the consumption energy consumed by the body movement of the user can be calculated assuming that a running exercise is included in the walking exercise.

## Patentansprüche

1. Erfassungsvorrichtung für Körperbewegungen, die Folgendes umfasst:
eine Körperbewegungsdaten-Erfassungseinheit zum Erfassen von Körperbewegungsdaten in Bezug auf eine Körperbewegung eines Anwenders, die durch das Bewegen der Erfassungsvorrichtung für Körperbewegungen erzeugt werden,
eine Unterscheidungseinheit für Körperbewegungen, die dafür angeordnet ist, auf der Grundlage der Körperbewegungsdaten zu unterscheiden, ob die Körperbewegung eine fortlaufende Gehübung oder eine andere Übung als die fortlaufende Gehübung ist, und
eine Berechnungseinheit, angeordnet für
das Berechnen einer Verbrauchsenergie während der Gehübung unter Verwendung einer ersten Berechnungsformel auf der Grundlage der Körperbewegungsdaten der Körperbewegung, die durch die Unterscheidungseinheit für Körperbewegungen unter den Körperbewegungsdaten als die Gehübung unterschieden wird, wobei die erste Berechnungsformel eine Formel zum Berechnen der durch die Gehübung verbrauchten Verbrauchsenergie ist,
das Berechnen einer Verbrauchsenergie zu der Zeit einer anderen Übung als der Gehübung unter Verwendung einer zweiten Berechnungsformel auf der Grundlage der Körperbewegungsdaten der Körperbewegung, die durch die Unterscheidungseinheit für Körperbewegungen unter den Körperbewegungsdaten als die andere Übung als die Gehübung unterschieden wird, wobei die zweite Berechnungsformel eine Formel zum Berechnen der durch die anderen Übungen als die Gehübung verbrauchten Verbrauchsenergie ist, und
das Berechnen einer Verbrauchsenergie durch die Körperbewegung des Anwenders durch Addieren dieser Verbrauchsenergien unter den Körperbewegungsdaten durch die Unterscheidungseinheit für Körperbewegungen,
wobei die Körperbewegungsdaten eine Differenz zwischen einem oberen Spitzenwert und einem unteren Spitzenwert einer Körperbewegungsstärke bei jeder bestimmten vergangenen Zeit und den Körperbewegungsabstand einschließen und
wobei die Unterscheidungseinheit für Körperbewegungen dafür angeordnet ist, die Körperbewegung des Anwenders als eine Gehübung zu unterscheiden, wenn die Differenz zwischen einem oberen Spitzenwert und einem unteren Spitzenwert der Körperbewegungsstärke bei jeder bestimmten vergangenen Zeit einen ersten Schwellenwert überschreitet, der als ein Schwellenwert der Differenz zwischen einem oberen Spitzenwert und einem unteren Spitzenwert der Körperbewegungsstärke bei jeder bestimmten vergangenen Zeit innerhalb eines ersten vorbestimmten Zeitraums definiert ist, und die Körperbewegung des Anwenders als die fortlaufende Gehübung zu unterscheiden, wenn die Anzahl der Male, wenn die Unterscheidungseinheit für Körperbewegungen die Körperbewegung des Anwenders als eine Gehübung unterscheidet, innerhalb eines zweiten vorbestimmten Zeitraums einen zweiten Schwellenwert überschreitet, der als ein Schwellenwert der Anzahl der Male, wenn die Unterscheidungseinheit für Körperbewegungen die Körperbewegung des Anwenders als eine Gehübung unterscheidet, innerhalb eines zweiten vorbestimmten Zeitraums definiert ist.

2. Erfassungsvorrichtung für Körperbewegungen nach Anspruch 1, die einen durch die Körperbewegung erzeugten Beschleunigungswert umfasst, wobei die Unterscheidungseinheit für Körperbewegungen einen Beschleunigungssensor einschließt, der dafür angeordnet ist, unterschiedliche Ausgangswerte entsprechend dem Beschleunigungswert auszugeben.

3. Erfassungsvorrichtung für Körperbewegungen nach Anspruch 1 oder 2, die ferner eine Erfassungseinheit für biologische Daten umfasst, die dafür angepasst ist, biologische Daten des Anwenders zu erfassen, wobei die Berechnungseinheit dafür angeordnet ist, die durch die Körperbewegung des Anwenders verbrauchte Verbrauchsenergie unter Verwendung einer Berechnungsformel zu berechnen, welche die durch die Erfassungseinheit für biologische Daten erfassten biologischen Daten und die Körperbewegungsdaten als Parameter hat.

4. Erfassungsvorrichtung für Körperbewegungen nach Anspruch 3, die derart angeordnet ist, dass die Berechnung der Verbrauchsenergie während der Gehübung erreicht wird durch die Verwendung einer Berechnungsformel, die wenigstens Parameter wie beispielsweise ein Körpergewicht als die biologischen Daten und einen Koeffizienten und eine Anzahl von Schritten entsprechend dem Körperbewegungsabstand des Anwenders als die Körperbewegungsdaten einschließt.

5. Erfassungsvorrichtung für Körperbewegungen nach Anspruch 3 oder Anspruch 4, die derart angeordnet ist, dass die Berechnung der Verbrauchsenergie während anderer Übungen als der Gehübung erreicht wird durch die Verwendung der Berechnungsformel, die wenigstens Parameter wie beispielsweise ein Körpergewicht und eine magere Körpermasse als die biologischen Daten und Daten in Bezug auf einen durch die Körperbewegung erzeugten Beschleunigungswert als die Körperbewegungsdaten einschließt.

6. Erfassungsvorrichtung für Körperbewegungen nach einem der Ansprüche 1 bis 5, die derart angeordnet ist, dass die durch die Körperbewegung des Anwenders verbrauchte Verbrauchsenergie unter der Annahme berechnet werden kann, dass eine Laufübung in der Gehübung eingeschlossen ist.

## Revendications

1. Appareil de détection de mouvement de corps comprenant :
une unité d'acquisition de mouvement de corps conçue pour acquérir des données de mouvement de corps relatives à un mouvement de corps d'un utilisateur qui sont générées en déplaçant l'appareil de détection de mouvement de corps,
une unité de discrimination de mouvement de corps conçue pour discriminer si le mouvement de corps est un exercice de marche continue ou un exercice autre que l'exercice de marche continue sur la base des données de mouvement de corps ; et
une unité de calcul conçue pour
calculer une énergie de consommation pendant l'exercice de marche à l'aide d'une première formule de calcul sur la base des données de mouvement de corps du mouvement de corps qui est discriminé comme l'exercice de marche parmi les données de mouvement de corps par l'unité de discrimination de mouvement de corps, la première formule de calcul étant une formule permettant de calculer l'énergie de consommation consommée par l'exercice de marche,
calculer une énergie de consommation au moment d'un exercice autre que l'exercice de marche en utilisant une seconde formule de calcul sur la base des données de mouvement de corps du mouvement de corps qui est discriminé comme l'exercice autre que l'exercice de marche parmi les données de mouvement de corps par l'unité de discrimination de mouvement de corps, la seconde formule de calcul étant une formule permettant de calculer l'énergie de consommation consommée par les exercices autres que l'exercice de marche, et
calculer une énergie de consommation par le mouvement de corps de l'utilisateur en ajoutant ces énergies de consommation provenant des données de mouvement de corps par l'unité de discrimination de mouvement de corps ;
dans lequel les données de mouvement de corps comprennent une différence entre une valeur de crête supérieure et une valeur de crête inférieure d'une force de mouvement de corps à chaque certain temps écoulé et le degré de mouvement de corps, et
l'unité de discrimination de mouvement de corps est conçue pour discriminer le mouvement de corps de l'utilisateur comme étant un exercice de marche lorsque la différence entre une valeur de crête supérieure et une valeur de crête inférieure de la force de mouvement de corps à chaque certain temps écoulé dépasse une première valeur seuil définie en tant que valeur seuil de la différence entre une valeur de crête supérieure et une valeur de crête inférieure de la force de mouvement de corps à chaque certain temps écoulé dans une première période prédéterminée, et pour discriminer le mouvement de corps de l'utilisateur comme étant l'exercice de marche continue lorsque le nombre de fois que l'unité de discrimination de mouvement de corps discrimine le mouvement de corps de l'utilisateur comme étant une marche dans une seconde période prédéterminée dépasse une seconde valeur seuil définie en tant que valeur seuil du nombre de fois que l'unité de discrimination de mouvement de corps différencie le mouvement de corps de l'utilisateur comme étant un exercice de marche dans une seconde période prédéterminée.

2. Appareil de détection de mouvement de corps selon la revendication 1, comprenant une valeur d'accélération générée par le mouvement de corps, dans lequel l'unité de discrimination de mouvement de corps comprend un capteur d'accélération conçu pour émettre en sortie différentes valeurs de sortie selon la valeur d'accélération.

3. Appareil de détection de mouvement de corps selon les revendications 1 ou 2, comprenant en outre une unité d'acquisition de données biologiques qui est adaptée pour acquérir des données biologiques de l'utilisateur, dans lequel l'unité de calcul est conçue pour calculer l'énergie de consommation consommée par le mouvement de corps de l'utilisateur en utilisant une formule de calcul ayant les données biologiques acquises par l'unité d'acquisition de données biologiques et les données de mouvement de corps en tant que paramètres.

4. Appareil de détection de mouvement de corps selon la revendication 3, conçu de telle sorte que le calcul de l'énergie de consommation pendant l'exercice de marche est réalisé en utilisant une formule de calcul comprenant des paramètres au moins tels qu'un poids corporel en tant que données biologiques et un coefficient et un nombre de pas correspondant au degré de mouvement de corps de l'utilisateur en tant que données de mouvement de corps.

5. Appareil de détection de mouvement de corps selon la revendication 3 ou la revendication 4, conçu de telle sorte que le calcul de l'énergie de consommation pendant les exercices autres que l'exercice de marche est réalisé en utilisant la formule de calcul comprenant des paramètres au moins tels que le poids corporel et une masse corporelle maigre en tant que données biologiques et des données relatives à une valeur d'accélération générée par le mouvement de corps en tant que données de mouvement de corps.

6. Appareil de détection de mouvement de corps selon l'une quelconque des revendications 1 à 5, conçu de telle sorte que l'énergie de consommation consommée par le mouvement de corps de l'utilisateur peut être calculée en supposant qu'un exercice de course est inclus dans l'exercice de marche.
